# FASCICULE DE BREVET EUROPEEN

(11) **EP 1 241 998 B1**
(45) Date de publication et mention de la délivrance du brevet: **10.11.2004**
(21) Numéro de dépôt: 99964747.2
(22) Date de dépôt: 30.12.1999
(51) Int. Cl.: A61B 19/02

(54) **DISPOSITIF POUR LA MANIPULATION ET L'IDENTIFICATION DE PETITS IMPLANTS**
VORRICHTUNG ZUR HANDHABUNG UND ZUR IDENTIFIZIERUNG KLEINER IMPLANTATE
DEVICE FOR HANDLING AND IDENTIFYING SMALL IMPLANTS

(43) Date de publication de la demande: 25.09.2002
(73) Titulaire: Biotech International (SARL), 13300 Salon de Provence (FR)
(72) Inventeur: IMPELLIZZERI, Frédéric, F-13300 Salon de Provence (FR)
(74) Mandataire: Marek, Pierre
(86) Numéro de dépôt international: PCT/FR1999/003323
(87) Numéro de publication internationale: WO 2001/049198

(56) Documents cités:
- EP-A- 0 736 464
- WO-A-00/07510
- WO-A-99/42049
- DE-B- 1 074 819
- DE-U- 29 705 944
- FR-A- 2 785 033
- US-A- 2 824 651
- US-A- 4 253 830

## Description

La présente invention concerne un dispositif identificateur pour la manipulation et l'identification de petits implants (vis ou autres) utilisés en chirurgie restauratrice (chirurgie orthopédique, chirurgie maxillo-faciale, ostéosynthèse, etc.).

Dans une application très intéressante, le dispositif identificateur selon l'invention permet d'identifier et de suivre l'itinéraire d'un implant de dimension très réduite (par exemple de l'ordre de 1 à 2 mm), de sa fabrication à son implantation sur un patient.

Depuis une date récente, une directive de la Commission des Communautés Européennes impose la traçabilité, c'est à dire l'identification et le suivi de tout matériel à usage chirurgical destiné à une mise en place in vivo, de sa fabrication à son utilisation finale, par exemple jusqu'à son implantation dans le corps d'un patient. Chaque produit sortant de l'usine de fabrication doit ainsi être identifié notamment par un numéro de lot qui doit pouvoir être lu et relevé à tout moment. Ainsi, si un implant s'avérait défectueux pour une quelconque raison, le numéro d'identification doit permettre ou tout au moins faciliter la détermination de l'origine de la défaillance.

D'autre part, il est grandement souhaitable que les chirurgiens ou leurs assistants pratiquant en bloc opératoire puissent, d'une part, facilement saisir les petits implants tels que les vis utilisées pour la coaptation de fragments d'os ou autres, et, d'autre part, lire et reporter le numéro de ceux-ci sur les dossiers des patients. De plus, dès qu'un implant est stérilisé, la manipulation doit être la plus indirecte possible (tout en restant aisée) pour ne pas contaminer ledit implant.

Le matériel chirurgical de dimension réduite parvenant au bloc opératoire et destiné à l'implantation sur un patient doit donc être aisément identifiable et de manipulation facile sans que sa stérilisation puisse s'en trouver compromise.

La chirurgie actuelle emploie dans certains domaines (orthopédie, chirurgie maxillo-faciale, ostéosynthèse, interventions sur les petits os, les petits fragments ou les extrémités du squelette, ...) du matériel de très petite taille (inférieure à 2 mm) dont la destination est l'implantation dans le corps d'un patient.

Actuellement, le matériel chirurgical destiné aux implantations « in vivo » est livré aux établissements hospitaliers :
- soit déjà stérilisé et conditionné dans des boîtes stériles lesquelles sont amenées fermées en bloc opératoire pour les implantations ;
- soit non stérilisé, ensuite de quoi il est réparti dans des racks ou plateaux au moyen desquels il est ensuite stérilisé en autoclave, avant d'être amené en bloc opératoire.

Dans le premier cas qui est une solution très coûteuse, il est simple de placer dans les boîtes, avant la stérilisation, des étiquettes comportant le numéro du lot, de sorte que lors de l'ouverture desdites boîtes en vue de l'utilisation du matériel stérilisé, de collecter les étiquettes et de les placer dans les dossiers des patients.

Cependant, ce mode de conditionnement des implants impose l'utilisation d'un volume important de boîtes, ce qui augmente le coût du transport et complique le stockage et les manipulations. Un autre inconvénient de ce mode de conditionnement est que l'on est parfois conduit à ouvrir plusieurs boîtes pour obtenir les pièces nécessaires, en annulant la stérilisation des implants non utilisés.

Dans le second cas, la caractérisation des implants permettant leur identification et la traçabilité imposée par la directive européenne, est très difficile à réaliser.

En effet, le numéro d'identification attribué à chaque implant ou à chaque lot d'implants comporte le plus souvent une succession de plusieurs chiffres et/ou lettres, et la gravure de ce numéro directement sur les implants, si elle est possible et d'usage courant pour les implants relativement grands, devient quasiment irréalisable sur les implants de dimension très réduite, par manque de place. A supposer qu'une microgravure soit possible dans certains cas, la lecture aisée de ce numéro par exemple par le chirurgien ou par l'un de ses assistants, en cours d'opération demeurerait impossible, que ce soit à l'oeil nu ou à l'aide d'une loupe. Une autre possibilité pourrait être d'utiliser la petite place disponible sur chaque implant pour y graver des signes de reconnaissance occupant une très petite surface, sous forme de code mais là encore, la lecture de ce code par le personnel médical sans un matériel spécialisé pour le décodage est impossible.

EP-A2-0 736 464 montre les caractéristiques du preambule de la revendication 1.

L'invention a notamment pour but de remédier aux inconvénients susmentionnés.

Selon l'invention cet objectif est atteint grâce à un dispositif identificateur selon la revendication 1, comprenant un petit implant et un support identificateur appariés, ce dernier comportant un corps exécuté, au moins en partie, dans un matériau présentant une capacité de déformation élastique, ce corps comprenant un logement rétenteur, par exemple un logement constricteur, de forme appropriée à celle de l'implant de petite dimension apparié audit support, et dans lequel est logé, au moins en partie, cet implant, ledit support comportant encore un moyen de caractérisation permettant son identification et, par conséquent, la reconnaissance de l'implant apparié audit support identificateur.

On comprend que, selon l'invention, chaque implant est clipsé dans un support identificateur qui présente, par exemple sur l'une de ses faces et de manière facilement lisible, l'inscription des informations nécessaires à l'identification de l'implant qu'il porte. Les couples support identificateur/implant peuvent être livrés en vrac, puis placés dans des casiers ou containers pour la stérilisation à la vapeur à 138°C dans un four autoclave. Les couples support identificateur /implant peuvent être stérilisés autant de fois qu'il est nécessaire sans perdre les informations identifiant l'implant. Le dispositif selon l'invention facilite ainsi grandement l'identification et la manipulation des petits implants chirurgicaux. Le dispositif identificateur selon l'invention permet également le maintien du matériel en place dans le container, notamment lors du transport de ce dernier.

Un avantage très important du dispositif identificateur selon l'invention est qu'il permet de ménager, sur la surface externe du support, une plage suffisamment grande pour que l'on puisse y graver un numéro ou autre repère d'identification aisément lisible, en per opératoire, par les chirurgiens ou leurs assistants, c'est-à-dire au moment de l'implantation des implants. On comprend que cette possibilité facilite grandement l'obtention de la traçabilité imposée par les textes réglementaires.

Le dispositif identificateur selon l'invention permet également et de façon avantageuse la manipulation de l'implant au moyen d'instruments stériles, sans toucher et donc sans contaminer celui-ci, par exemple lors de la mise en place des implants reçus en vrac dans les casiers.

Suivant une autre disposition caractéristique le support du dispositif identificateur selon l'invention comporte une fente latérale débouchant dans le logement rétenteur dudit support, les bords de cette fente pouvant être écartés ou rapprochés grâce à l'élasticité du matériau.

De la sorte, l'implant ou autre petit objet peut être engagé facilement et quasi instantanément dans le logement constricteur du support ou dégagé tout aussi facilement et rapidement de ce logement.

Suivant une autre disposition caractéristique, le support du dispositif identificateur comporte un évidement latéral disposé dans l'alignement du logement constricteur et dans laquelle débouche celui-ci.

Grâce à cette disposition, l'implant tel que vis ou autre peut être saisi et dégagé facilement de son support apparié au moyen d'un instrument approprié tel qu'une pince ou brucelles précédemment stérilisée, ce qui contribue à la conservation de l'état stérile dudit implant.

Les buts, caractéristiques et avantages ci-dessus, et d'autres encore, ressortiront mieux de la description qui suit et des dessins annexés dans lesquels :
- la figure 1 est une vue en perspective avec arrachement partiel d'un premier exemple de réalisation du dispositif identificateur de petits implants selon l'invention ;
- la figure 2 est une vue de face de l'extrémité de la portion du support du dispositif identificateur pourvue du logement constricteur ;
- la figure 3 est une vue en coupe axiale dudit support du dispositif identificateur, considérée suivant la ligne 3-3 de la figure 2 ;
- la figure 4 est une vue en perspective du support du dispositif identicateur, considéré du côté muni de la gravure d'identification ;
- la figure 5 est une vue en plan d'une autre forme de réalisation du dispositif identificateur selon l'invention ;
- la figure 6 est une vue en coupe longitudinale selon la ligne 6-6 de la figure 5;
- la figure 7 est une vue en perspective du support du dispositif identificateur représenté aux figures 5 et 6 ;
- la figure 8 est une vue de face de l'une des extrémités de ce support ;

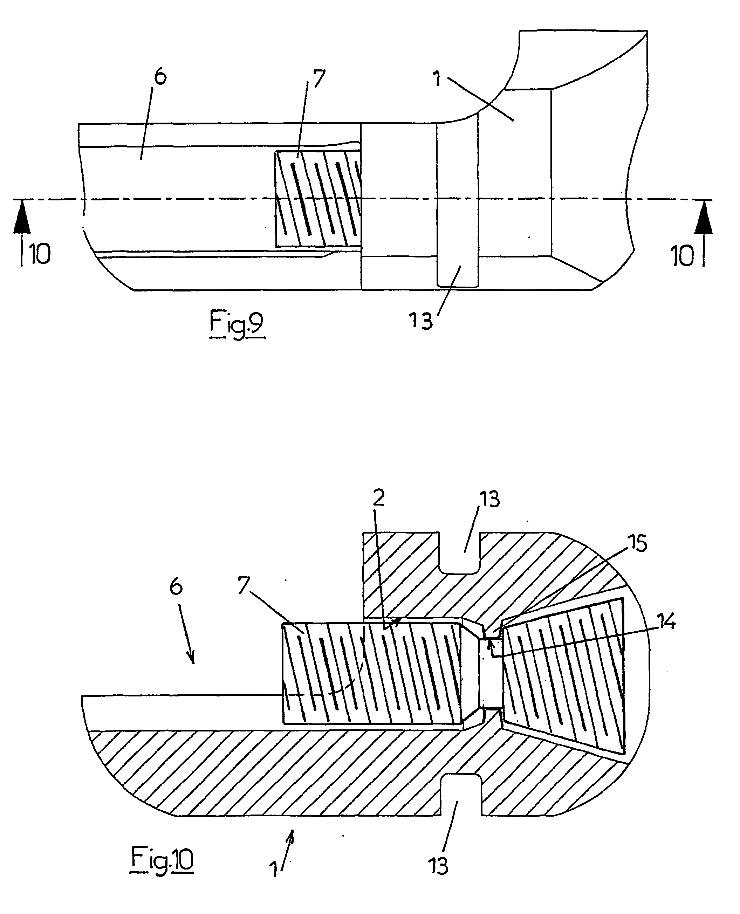

- la figure 9 est une vue de côté montrant l'appariement d'un support identique et d'une vis présentant des caractéristiques différentes de celle représentée aux figures 5 et 6 ;
- la figure 10 est une vue en coupe longitudinale selon la ligne 10-10 de la figure 9 ;
- la figure 11 est une vue en coupe transversale d'un plateau dans les gorges longitudinales duquel sont positionnés des couples support identificateur/implant selon l'invention.

On se réfère auxdits dessins pour décrire deux exemples de réalisation intéressants, bien que nullement limitatifs, du dispositif identificateur selon l'invention.

Ce dispositif comprend un petit implant 7 et un support identificateur 1 appariés.

Le petit implant 7 peut être constitué par une vis d'ostéosynthèse utilisée en chirurgie orthopédique, en chirurgie maxillo-faciale, ou autres.

Le support identificateur comprend un corps 1 réalisé, en un matériau doté d'une capacité de déformation élastique et capable de supporter les températures élevées de stérilisation en autoclaves, par exemple en matériau élastomère. Il peut être exécuté en totalité ou au moins en partie dans un tel matériau. Il peut s'agir d'un élastomère thermoplastique à base de polyéthylène et de butylène (groupe central) et de polystyrène (terminaisons) par exemple du genre connu sous la dénomination "KRATON" (Marque Déposée).

Le corps 1 présente une forme allongée et il comporte un logement rétenteur 2 dont l'axe est orienté longitudinalement selon une disposition avantageuse de l'invention, mais qui pourrait avoir d'autres orientations, suivant les applications de celle-ci.

De manière préférée, le logement rétenteur 2 s'étend sur une partie seulement de la longueur du corps 1. Dans cette partie, le corps 1 est pourvu d'une fente longitudinale 3 s'ouvrant à l'extérieur, c'est-à-dire d'une fente orientée parallèlement à l'axe du logement rétenteur 2 dans laquelle débouche ladite fente. On comprend qu'en raison de l'élasticité du matériau dans lequel est exécuté le corps 1, les bords opposés 4a, 4b de la fente 3 peuvent être éloignés l'un de l'autre, par application d'une force d'écartement, pour permettre le positionnement d'un petit implant 7 dans ledit logement, ou rapprochés, par effet d'élasticité, de façon à maintenir en place ledit implant dans le logement 2.

De façon avantageuse, le logement rétenteur 2 présente une forme complémentaire de celle de l'implant auquel il est apparié. Ce logement peut avoir une conformation permettant de retenir l'implant, avec un petit jeu ou sans jeu. Alternativement ou complémentairement, le logement rétenteur 2 peut être constricteur, c'est-à-dire apte à exercer une action de serrage autour de l'implant, par l'effet d'élasticité du matériau. Dans ce cas, au repos, le logement présente un diamètre très légèrement inférieur à celui de l'implant.

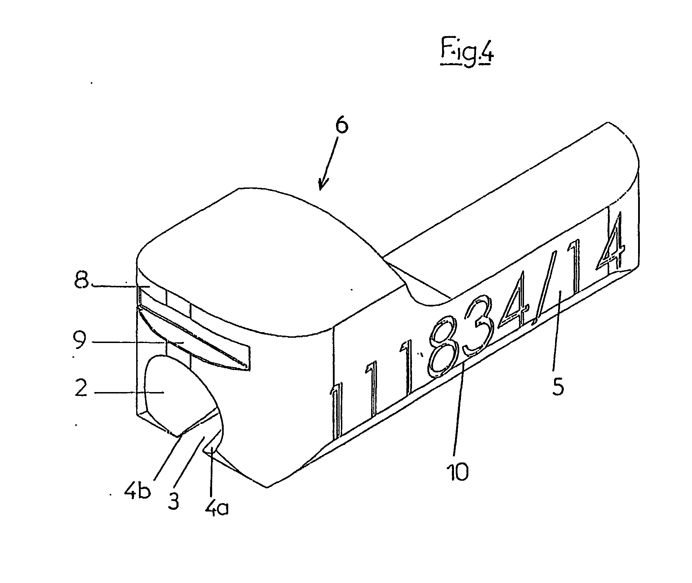

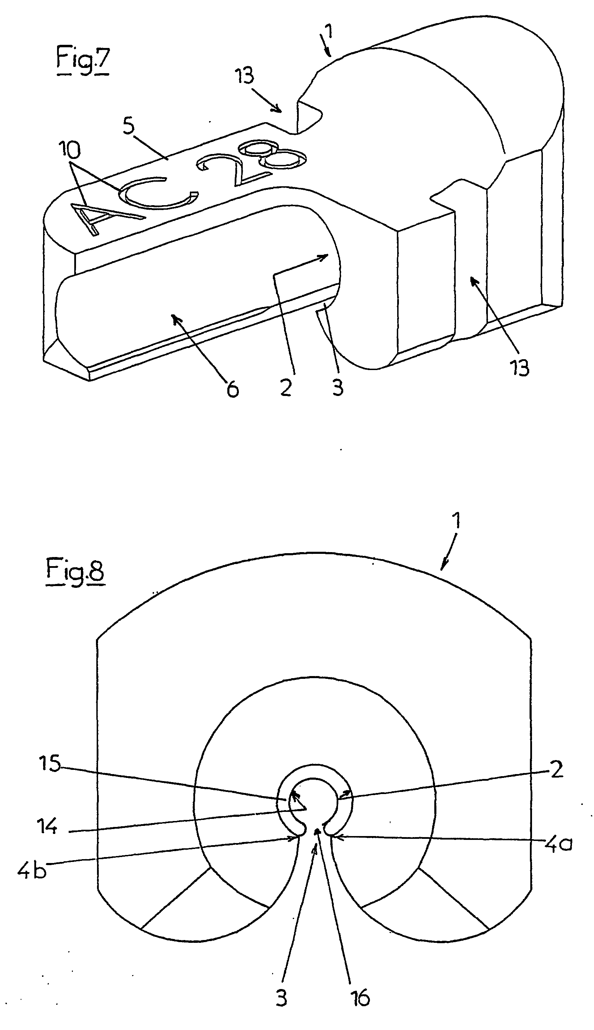

Latéralement (figure 4) ou sur le dessus (figure 7), le corps présente une surface 5, de préférence plane et lisse sur laquelle est gravé ou autrement rapporté, un repère d'identification 10, par exemple constitué par un numéro de lot, aisément lisible. Suivant son emplacement, cette surface peut occuper une longueur correspondant approximativement à la longueur du corps 1 ou une longueur inférieure à celle-ci.
Lorsqu'un implant 7 a été positionné dans un support 1 individualisé par un repère d'identification 10, il forme avec celui-ci un ensemble ou dispositif identificateur dont l'intégrité ne sera détruite qu'au moment de l'utilisation de l'implant par le chirurgien.
De manière intéressante, le support identificateur comporte un évidement ouvert 6 s'étendant longitudinalement, sur une longueur sensiblement égale à la demi-longueur du corps 1. Cet évidement 6 est disposé dans le prolongement du logement 2 lequel débouche dans ledit évidement. Ce dernier permet de saisir facilement l'implant 7 retenu dans le corps 1, avec une pince chirurgicale, pour le dégager de son support. Il permet en outre une bonne exposition de l'implant à la source de stérilisation.

Les implants et leurs supports ainsi appariés peuvent être engagés par pression dans les logements que présentent les plateaux utilisés pour la stérilisation et la présentation en bloc opératoire. Ils restent positionnés à leurs emplacements durant leur transport par l'effet de la pression qu'exercent les supports sur les parois de leurs logements sous l'action de l'élasticité du matériau dans lequel sont exécutés lesdits supports.

On a représenté sur la figure 11, un plateau 11 comportant deux gorges longitudinales 12 dans lesquelles sont positionnés et maintenus deux couples supports identificateurs 1/ vis 7 appariés, par exemple, en vue de leur utilisation en bloc opératoire. Les gorges 12 du plateau 11 présentent une largeur L très légèrement inférieure à celle des dispositifs identificateurs, de sorte que ces derniers puissent être engagés sous une légère pression entre les parois 12a desdites gorges et se trouver ensuite maintenus en position dans ces dernières grâce à l'élasticité de l'élastomère dans lequel le corps 1 est exécuté.

Selon une autre disposition caractéristique, le corps 1 du support identificateur présente, extérieurement, un moyen destiné à faciliter la préhension du dispositif identificateur au moyen d'un instrument de la procédure opératoire (précelles chirurgicales, par exemple).

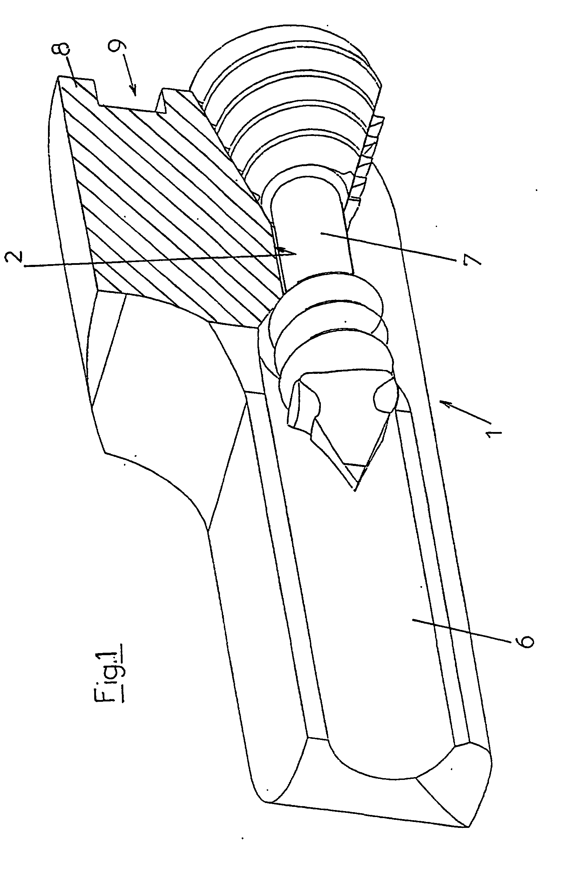

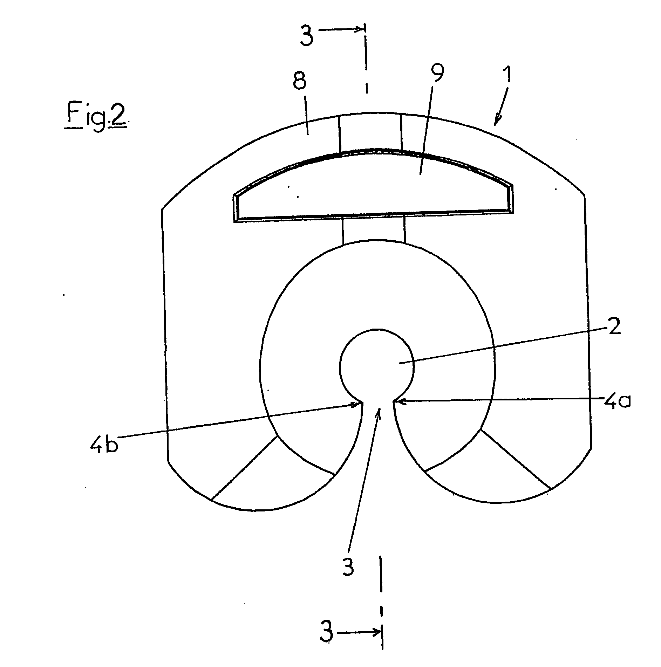

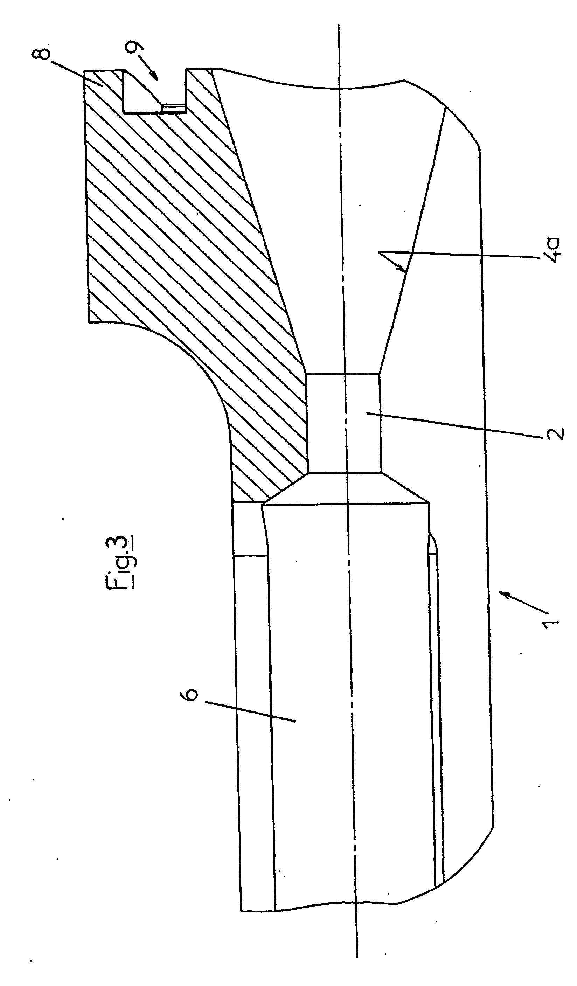

Selon le mode d'exécution représenté aux figures 1 à 4, le corps 1 comporte une languette de préhension 8 laquelle résulte, par exemple, de la prévision d'une fente transversale 9 que présente l'extrémité libre de la partie du corps 1 qui délimite le logement 2.

En considérant, comme indiqué précédemment, que les ensembles support identificateur 1/implant 7, appariés sont amenés en bloc opératoire dans des plateaux dans lesquels ils se trouvent positionnés de manière déterminée par pincement, la languette 8 permet de saisir l'ensemble incluant l'implant désiré et de le dégager de son logement au moyen d'un instrument de chirurgie approprié tel que précelles.

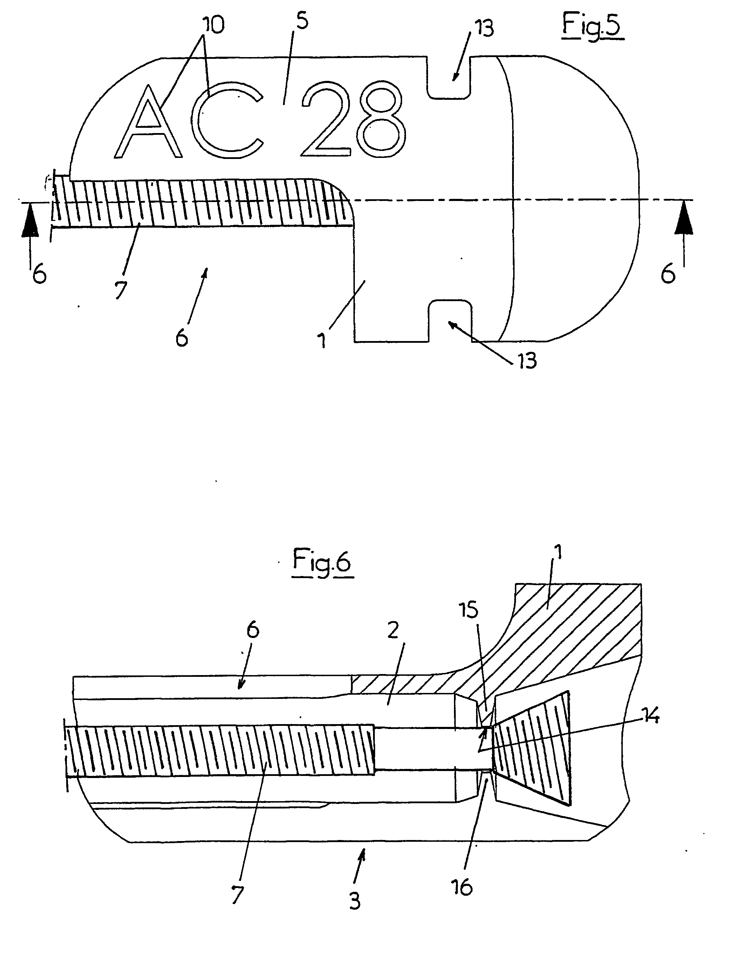

Suivant le mode d'exécution illustré aux figures 5 à 8, les moyens de préhension du dispositif identificateur 1-7 sont constitués par deux gorges parallèles 13 que présentent, symétriquement, les faces latérales opposées de la partie du corps 1 dans laquelle est ménagé le logement 2, ces gorges étant disposées de chaque côté de la fente 3 débouchant dans ce dernier. En engageant les becs de la pince de préhension dans les gorges 13, il est facile de dégager le dispositif du plateau et de retirer ensuite l'implant 7 du support 1, par exemple au moyen d'un autre instrument stérile approprié.

Selon une autre disposition caractéristique de l'invention, le logement 2 comporte, de préférence dans sa partie médiane, un étranglement 14 délimité par une lèvre souple circulaire 15. Cette lèvre souple et compressible présente une fente 16 communiquant avec la fente 3 du corps 1. La lèvre ou bague souple fendue 15 délimite un passage axial de diamètre nettement inférieur au diamètre de la portion restante du logement 2.

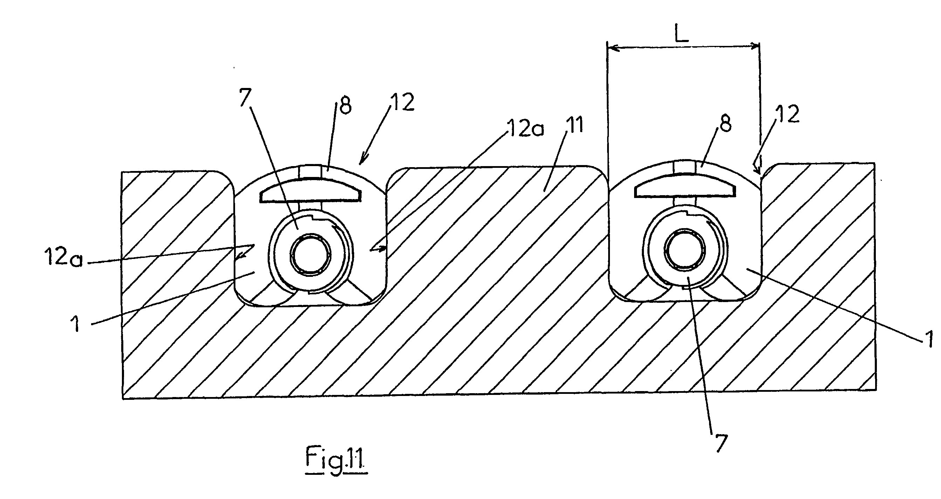

Grâce à cette disposition, des vis de diamètres différents ou autres petits implants peuvent être appariés à des supports identificateurs identiques, comme le montrent, par exemple, les figures 6 et 10, ce qui évite la fabrication et le stockage de plusieurs tailles de supports identificateurs 1.

## Revendications

1. Dispositif identificateur pour la manipulation et l'identification de petits implants utilisés pour la coaptation de fragments d'os et pour l'ostéosynthèse, constitué d'un implant de petite dimension et d'un support identificateur appariés, ce dernier comprenant un corps (1) exécuté, au moins en partie, dans un matériau présentant une capacité de déformation élastique, et muni d'un logement rétenteur (2) de forme appropriée à celle de l'implant (7) de petite dimension apparié audit support et dans lequel se trouve logé, au moins en partie, cet implant ; ledit corps (1) étant pourvu d'une fente (3) débouchant dans ledit logement (2) et ledit support comportant encore un moyen de caractérisation (5-10) permettant son identification et, par conséquent, la reconnaissance dudit implant apparié audit support identificateur, **caractérisé en ce que** le logement rétenteur (2) du support identificateur comporte un étranglement constricteur (14) délimité par une lèvre souple circulaire (15) et retenant l'implant de petite dimension (7) par une action de serrage s'exerçant autour de ce dernier.

2. Dispositif identificateur pour la manipulation et l'identification de petits implants utilisés pour la coaptation de fragments d'os et pour l'ostéosynthèse, selon la revendications 1, **caractérisé en ce que** la lèvre souple circulaire (15) est fendue et **en ce que** cette fente (16) communique avec la fente (3) du corps identificateur (1).

3. Dispositif identificateur pour la manipulation et l'identification de petits implants utilisés pour la coaptation de fragments d'os et pour l'ostéosynthèse, selon l'une des revendications 1 ou 2, **caractérisé en ce que** le logement rétenteur (2) s'étend sur une partie seulement de la longueur du corps (1) du support identificateur lequel comporte un évidement (6) ouvert à l'extérieur et disposé dans le prolongement du logement (2) lequel débouche dans ledit évidement.

4. Dispositif identificateur pour la manipulation et l'identification de petits implants utilisés pour la coaptation de fragments d'os et pour l'ostéosynthèse, selon l'une quelconque des revendications 1 à 3, **caractérisé en ce que** le support identificateur (1) présente, extérieurement, un moyen (8-9 ; 13) facilitant sa préhension à l'aide d'un instrument de la procédure opératoire.

5. Dispositif identificateur pour la manipulation et l'identification de petits implants utilisés pour la coaptation de fragments d'os et pour l'ostéosynthèse, selon la revendication 4, **caractérisé en ce que** les moyens destinés à faciliter sa préhension comprennent deux gorges parallèles (13) que présentent, symétriquement, les faces latérales opposées du corps (1) du support identificateur.

6. Dispositif identificateur pour la manipulation et l'identification de petits implants utilisés pour la coaptation de fragments d'os et pour l'ostéosynthèse, selon l'une quelconque des revendications 1 à 5, **caractérisé en ce que** ledit support identificateur est exécuté dans un matériau apte à supporter les températures de stérilisation en autoclave.

7. Dispositif identificateur pour la manipulation et l'identification de petits implants utilisés pour la coaptation de fragments d'os et pour l'ostéosynthèse, selon l'une quelconque des revendications 1 à 5, **caractérisé en ce que** le corps (1) du support identificateur comporte, extérieurement, une surface plane (5) portant un repère (10), par exemple constitué par un numéro de lot.

8. Dispositif identificateur pour la manipulation et l'identification de petits implants utilisés pour la coaptation de fragments d'os et pour l'ostéosynthèse selon l'une quelconque des revendications 1 à 7, ledit dispositif comprenant encore un plateau (11) muni de gorges (12) pour le positionnement desdits petits implants et de leurs supports identificateurs, **caractérisé en ce que** lesdites gorges (12) présentent une largeur (L) très légèrement inférieure à celle du support identificateur (1) de sorte que ces derniers puissent être engagés sous une légère pression manuelle dans lesdites gorges (12) et se trouver ensuite maintenus en position dans ces dernières grâce à l'élasticité du matériau dans lequel le corps (1) est exécuté.

## Patentansprüche

1. Identifizierungsvorrichtung für die Manipulation und Identifizierung von kleinen Implantaten, die zum Aufeinanderpassen von Knochenfragmenten und zur Osteosynthese verwendet werden, die aus einem Implantat mit kleiner Abmessung und einer Identifizierungs-Halterung besteht, die zusammenpassen, wobei die Letztere einen Körper (1) umfasst, der wenigstens teilweise aus einem Material ausgeführt ist, das eine Fähigkeit zur elastischen Verformbarkeit aufweist, und mit einer Rückhalteaufnahme (2) in einer Form ausgestattet ist, die derjenigen des Implantats (7) mit kleiner Abmessung entspricht, das mit der Halterung zusammenpasst, und in der dieses Implantat wenigstens teilweise gelagert ist; wobei dieser Körper (1) mit einem Schlitz (3) versehen ist, der in die Aufnahme (2) mündet, und wobei die Halterung noch ein Kennzeichnungsmittel (5-10) umfasst, das ihre Identifizierung gestattet und infolgedessen das Erkennen des mit der Identifizierungs-Halterung zusammenpassenden Implantats, **dadurch gekennzeichnet, dass** die Rückhalteaufnahme (2) der Identifizierungs-Halterung eine einschnürende Querschnittsverminderung (14) umfasst, die durch eine weiche kreisförmige Lippe (15) begrenzt wird und das Implantat (7) mit kleiner Abmessung durch eine Spannwirkung zurückhält, die um das Letztere ausgeübt wird.

2. Identifizierungsvorrichtung für die Manipulation und Identifizierung von kleinen Implantaten, die zum Aufeinanderpassen von Knochenfragmenten und zur Osteosynthese verwendet werden, nach Anspruch 1, **dadurch gekennzeichnet, dass** die weiche kreisförmige Lippe (15) geschlitzt ist, und dadurch, dass dieser Schlitz (16) mit dem Schlitz (3) des Identifizierungskörpers (1) in Verbindung steht.

3. Identifizierungsvorrichtung für die Manipulation und Identifizierung von kleinen Implantaten, die zum Aufeinanderpassen von Knochenfragmenten und zur Osteosynthese verwendet werden, nach einem der Ansprüche 1 oder 2, **dadurch gekennzeichnet, dass** die Rückhalteaufnahme (2) sich nur über einen Teil der Länge des Körpers (1) der Identifizierungs-Halterung erstreckt, welche eine Aussparung (6) umfasst, die nach außen offen ist und in der Verlängerung der Aufnahme (2) angeordnet ist, die in die Aussparung mündet.

4. Identifizierungsvorrichtung für die Manipulation und Identifizierung von kleinen Implantaten, die zum Aufeinanderpassen von Knochenfragmenten und zur Osteosynthese verwendet werden, nach einem der Ansprüche 1 bis 3, **dadurch gekennzeichnet, dass** die Identifizierungs-Halterung (1) an der Außenseite ein Mittel (6-9; 13) aufweist, das ihr Greifen mit Hilfe eines Instruments des Operationsverfahrens erleichtert.

5. Identifizierungsvorrichtung für die Manipulation und Identifizierung von kleinen Implantaten, die zum Aufeinanderpassen von Knochenfragmenten und zur Osteosynthese verwendet werden, nach Anspruch 4, **dadurch gekennzeichnet, dass** die Mittel, die zum Erleichtern des Greifens bestimmt sind, zwei parallele Vertiefungen (13) umfassen, die symmetrisch die entgegengesetzten Seitenflächen des Körpers (1) der Identifizierungs-Halterung aufweisen.

6. Identifizierungsvorrichtung für die Manipulation und Identifizierung von kleinen Implantaten, die zum Aufeinanderpassen von Knochenfragmenten und zur Osteosynthese verwendet werden, nach einem der Ansprüche 1 bis 5, **dadurch gekennzeichnet, dass** die Identifizierungs-Halterung aus einem Material ausgeführt ist, das geeignet ist, die Sterilisierungstemperaturen im Autoklaven auszuhalten.

7. Identifizierungsvorrichtung für die Manipulation und Identifizierung von kleinen Implantaten, die zum Aufeinanderpassen von Knochenfragmenten und zur Osteosynthese verwendet werden, nach einem der Ansprüche 1 bis 5, **dadurch gekennzeichnet, dass** der Körper (1) der Identifizierungs-Halterung an der Außenseite eine ebene Fläche (5) umfasst, die eine Markierung (10) trägt, die beispielsweise aus der Chargennummer besteht.

8. Identifizierungsvorrichtung für die Manipulation und Identifizierung von kleinen Implantaten, die zum Aufeinanderpassen von Knochenfragmenten und zur Osteosynthese verwendet werden, nach einem der Ansprüche 1 bis 7, wobei die Vorrichtung des Weiteren eine Platte (11) umfasst, die mit Vertiefungen (12) für die Positionierung der kleinen Implantate und ihrer Identifizierungs-Halterungen ausgestattet ist, **dadurch gekennzeichnet, dass** die Vertiefungen (12) eine Breite (L) aufweisen, die sehr geringfügig kleiner als diejenige der Identifizierungs-Halterung (1) ist, so dass diese Letzteren unter einem leichten manuellen Druck in den Vertiefungen (12) zum Eingriff gebracht werden können und anschließend dank der Elastizität des Materials, aus dem der Körper (1) ausgeführt ist, in diesen Letzteren gehalten werden.

## Claims

1. Identifier device for the manipulation and identification of small implants used for the setting of fragments of bone and for osteosynthesis, consisting of a paired implant of small dimensions and an identifier support, the latter comprising a body (1) made, at least in part, of a material having the capacity of elastic deformation, and provided with a retainer mounting (2) of a shape appropriate to that of the implant (7) of small dimensions, paired to said support and in which this implant is located, at least in part, said body (1) being provided with a slot (3) opening into said mounting (2) and said support also comprising a characterisation means (5-10) allowing for its identification, and, consequently, recognition of said implant paired to said identifier, **characterised in that** the retainer mounting (2) of the identifier support comprises a constrictor choke (14) delimited by a flexible circular lip (15) and retaining the small-dimension implant (7) by a locking action imposed around the latter.

2. Identifier device for the manipulation and identification of small implants used for the setting of fragments of bone and for osteosynthesis according to Claim 1, **characterised in that** the flexible circular lip (15) is slotted, and that this slot (16) communicates with the slot (3) of the identifier body (1).

3. Identifier device for the manipulation and identification of small implants used for the setting of fragments of bone and for osteosynthesis according to any one of Claims 1 or 2, **characterised in that** the retainer mounting (2) extends over only a part of the length of the body (1) of the identifier support, which comprises a hollow (6) open to the outside and arranged in the extension of the mounting (2), which opens into said hollow.

4. Identifier device for the manipulation and identification of small implants used for the setting of fragments of bone and for osteosynthesis according to any one of Claims 1 to 3, **characterised in that** the identifier support (1) presents on its exterior means (8-9; 13) which facilitate its being gripped with the aid of a surgical instrument.

5. Identifier device for the manipulation and identification of small implants used for the setting of fragments of bone and for osteosynthesis according to Claim 4, **characterised in that** the means intended to facilitate its being gripped comprise two parallel throats (13), which present, symmetrically, lateral opposed faces of the body (1) of the identifer support.

6. Identifier device for the manipulation and identification of small implants used for the setting of fragments of bone and for osteosynthesis according to any one of Claims 1 to 5, **characterised in that** the said identifier support is made of a material capable of supporting the temperatures of sterilisation in an autoclave.

7. Identifier device for the manipulation and identification of small implants used for the setting of fragments of bone and for osteosynthesis according to any one of Claims 1 to 5, **characterised in that** the body (1) of the identifier support comprises, on its outside, a flat surface (5) carrying a reference mark (10), comprising, for example, a batch number.

8. Identifier device for the manipulation and identification of small implants used for the setting of fragments of bone and for osteosynthesis according to any one of Claims 1 to 7, said device also comprising a flat surface (11) provided with throats (12) for the positioning of said small implants and their identifier supports, **characterised in that** said throats (12) have a width (L) very slightly smaller than that of the identifier support (1) in such a way that these latter can be engaged under light manual pressure into said throats (12) and are then maintained in position in the latter thanks to the elasticity of the material of which the body (1) is made.
